# FASCICULE DE BREVET EUROPEEN

(11) **EP 2 627 747 B1**
(45) Date de publication et mention de la délivrance du brevet: **14.09.2016**
(21) Numéro de dépôt: 10765797.5
(22) Date de dépôt: 15.10.2010
(51) Int. Cl.: C11D 3/00, C11D 3/386, C11D 11/00, A61L 2/18, C11D 3/48

(54) **PROCEDE D'ELIMINATION DES BIOFILMS**
VERFAHREN ZUR ENTFERNUNG VON BIOFILMEN
METHOD FOR THE REMOVAL OF BIOFILMS

(43) Date de publication de la demande: 21.08.2013
(73) Titulaire: Realco SA, 1348 Louvain La Neuve (BE); Institut National de la Recherche Agronomique (INRA), 75338 Paris Cedex 07 (FR)
(72) Inventeur: BOELS, Gauthier, B-1040 Bruxelles (BE); BLACKMAN, Gordon, B-1380 Lasnes (BE); FAILLE, Christine, 59651 Villeneuve d'Ascq (FR); LEQUETTE, Yannick, F-62144 Mont saint Eloi (FR); CLARISSE, Martine, F-59390 Lys-lez-Lannoy (FR)
(74) Mandataire: Coulon, Ludivine
(86) Numéro de dépôt international: PCT/EP2010/065567
(87) Numéro de publication internationale: WO 2012/048758

(56) Documents cités:
- EP-A1- 2 243 821
- WO-A1-92/13807
- WO-A1-98/26807
- WO-A1-2007/095999
- WO-A1-2009/085743
- WO-A2-2006/031554
- US-A- 6 100 080
- US-A1- 2003 205 247

## Description

L'invention se rapporte au domaine de l'élimination des biofilms. Plus particulièrement, l'invention se rapporte à un procédé pour éliminer des biofilms.

L'hygiène prend une importance croissante dans l'industrie alimentaire, dans les hôpitaux, et en particulier dans la sphère chirurgicale, dans les installations de potabilisation et de désalinisation d'eau, dans le traitement des eaux de procédé, et en particulier dans les eaux utilisées dans les tours de refroidissement et dans les nécessités de la vie courante, par exemple les lentilles de contact. On observe fréquemment que, lors de la circulation d'eau sur un support, des microorganismes circulant librement dans l'eau peuvent adhérer à la surface. Ces microorganismes peuvent alors développer une matrice extracellulaire adhésive composée de substances polymères. Une communauté de microorganismes adhérés à une surface et englobée dans une telle matrice est appelée un biofilm. Généralement ces biofilms sont composés de bactéries.

On observe malheureusement que cette matrice est très résistante, et peut constituer une barrière pour des agents qui agiraient contre les microorganismes. Les traitements classiques à base de soude et/ou comportant différents biocides n'agissent pas de manière suffisamment efficace car ils ne pénètrent pas le biofilm sur toute son épaisseur ou sont inhibés par certaines molécules composant cette matrice. Le traitement n'est alors que partiellement efficace sur la surface supérieure du biofilm. En outre, ce dernier peut également piéger d'autres microorganismes, pathogènes notamment, que celui qui s'est installé initialement.

On connaît par WO98/26807 un procédé de traitement enzymatique d'un film biologique. Dans ce procédé, on met en contact le biofilm avec une composition de nettoyage comportant une ou plusieurs hydrolases pour éliminer ou libérer la couche de biofilm de la surface. Dans une seconde étape le biofilm est mis en contact avec une composition désinfectante bactéricide pour détruire les cellules bactériennes présentes dans le film. Cependant, l'utilisation simultanée de ces deux compositions est à l'origine d'un degré d'inactivation de certaines enzymes dans le mélange final. La rapidité et l'efficacité du nettoyage peuvent donc être améliorées en utilisant une composition où l'inactivation des enzymes est absente.

On connaît également du document US2003/0205247 une utilisation de solutions aqueuses contenant des enzymes pour le nettoyage de cuves de stockage ou de fermentation, contenant une ou plusieurs enzymes choisies parmi les suivantes : laccases, peroxydases, oxydoréductases, transférases, isomérases, lyases ou ligases et un agent épaississant avec un amplificateur de mousse. Le domaine d'action des solutions précitées est relativement étroit, propre à la brasserie, puisque les enzymes choisies et illustrées sont particulièrement connues pour être actives sur des polyphénols qui constituent principalement les résidus de fermentation, tannins et analogues. L'agent épaississant est un agent modifiant la viscosité et la thixotropie de la solution, utilisé dans le but de permettre une meilleure adhérence de la solution et/ou de la mousse sur les surfaces des cuves à traiter. Ces solutions ne conviennent pas pour le nettoyage d'installations différentes de cuves ou réservoirs (comprenant par exemple de nombreuses tubulures) et ne conviennent pas pour l'élimination d'autres types de microorganismes et donc pas pour une large gamme de biofilms.

Le document WO 92/13807 divulgue l'utilisation d'une composition permettant l'élimination de la biomasse et de biofilms sur des substrats dans des systèmes aqueux, c'est-à-dire dans des systèmes dans lesquels de l'eau est mise en circulation ou entreposée. Ce type de système souffre de la présence de biomasse et de biofilms alcalins ou acides dus au type d'organisme généralement présent dans ce type d'installation.

Pour résoudre ce problème, le document WO 92/13807 prévoit l'utilisation de polysaccharidases et/ou de protéases et de tensioactifs anioniques tels que du SDS ou DBS (respectivement du dodécylsulfate de sodium et de l'acide dodécylbenzènesulfonique) pour l'élimination de la biomasse et des biofilms.

Le document WO2006031554 porte également sur un procédé pour enlever, réduire ou déstructurer des biofilms présents sur une surface (substrat), ce procédé reposant sur l'application, sur le substrat, d'une alpha-amylase dérivant d'une bactérie.

Malheureusement, ce type de composition et ces procédés de l'état de la technique ne seront pas efficaces pour une large gamme de biofilms provoqués par divers microorganismes et présenteront une efficacité limitée pour l'élimination de biofilms.

Comme on peut le constater de ce qui précède, ces compositions et ces procédés sont toujours ciblés pour un type de biofilm ou un microorganisme ciblé et/ou pour une application particulière.

Il existe donc un besoin pour une composition et un procédé capables d'éliminer des biofilms, qui soient efficaces dans un délai raisonnable, agissent sur une large classe de biofilms produits par une large classe de microorganismes ou de groupes de microorganismes, qui ne soient pas nuisibles pour le support du biofilm, et agissent tant pour prévenir le développement de biofilms que sur des biofilms constitués depuis longtemps, et ayant atteint un stade de cohésion et de résistance important.

Pour résoudre ce problème, la présente invention procure une méthode pour l'élimination des biofilms présents sur un substrat caractérisée en ce qu'elle comporte les étapes suivantes de :
- a) mise à disposition d'un composant détergent comprenant un agent séquestrant ainsi qu'un agent mouillant et un agent dispersant et d'un composant enzymatique contenant au moins une protéase, au moins une laccase et au moins une polysaccharidase,
- b) mise en solution du composant détergent dans une phase aqueuse,
- c) mise en solution du composant enzymatique dans la solution formée à l'étape b) pour former une solution d'une composition pour l'élimination des biofilms présents sur un substrat, ladite solution formée présentant un pH compris approximativement entre 6,5 et 7,5,
   ou b') mise en solution du composant enzymatique dans une phase aqueuse,
- c') mise en solution du composant détergent dans la solution formée à l'étape b') pour former une solution pour l'élimination de biofilms, ladite solution formée présentant un pH compris approximativement entre 6,5 et 7,5.
- d) application de la solution de ladite la composition formée à l'étape c) ou c') sur le substrat pendant une période de temps prédéterminée, en particulier comprise entre 15 minutes et 4 heures
- e) une addition d'une solution basique suite à ladite application d) de la solution de ladite composition sur ledit substrat pendant ladite période de temps prédéterminée, de façon à effectuer un saut de pH jusqu'à environ 8 à 9.

Le procédé selon l'invention reposant sur la mise en oeuvre d'une composition comprenant un composant détergent et un composant enzymatique contenant au moins une protéase, au moins une laccase et au moins une polysaccharidase permet, de manière surprenante, d'améliorer significativement la rapidité et l'efficacité de l'élimination d'un biofilm tout en pouvant attaquer divers types de biofilms. Cette composition permet d'éliminer la totalité ou la quasi-totalité du biofilm et peut agir sur des biofilms même matures ou ayant un cycle de développement plus précoce et développés par de multiples espèces ou microorganismes différents.

A ce jour, il faut savoir qu'aucune composition efficace et qu'aucun procédé ne permet d'assurer l'élimination complète des biofilms présents dans les installations. Par exemple, dans le domaine de l'industrie alimentaire, les biofilms se forment de manière inévitable (au vu de la richesse du milieu environnant). Les biofilms présentent une activité de croissance cyclique comprenant une phase de croissance durant laquelle se produit l'accumulation des microorganismes et une phase de détachement durant laquelle des morceaux entiers de biofilms se détachent par érosion et sous l'effet de leur propre poids. Lorsqu'un industriel est confronté à ce phénomène, il devrait en réalité arrêter sa chaîne de production et effectuer différents cycles de lavages alternés à la soude, et avec de nombreux détergents et ou nettoyants chimiques et/ou enzymatiques puisqu'il n'existe pas de composition polyvalente. Or ceci représente de nombreuses heures de travail et de perte de rendement de l'installation.

Par conséquent, en pratique, la production n'est pas arrêtée et lorsque le biofilm est en phase de rupture, les lots produits sont contaminés et écartés jusqu'à ce que le niveau de contamination par les microorganismes des aliments produits soient à nouveau acceptable au vu des normes en vigueur. En outre, il n'est pas envisageable pour les industriels d'avoir en stock une solution détergente ou enzymatique particulière pour chaque microorganisme susceptible de contaminer sa chaîne de production.

C'est donc de manière très surprenante que le procédé selon la présente invention permet la mise à disposition d'une composition détergente parfaitement polyvalente qui permet d'éliminer un large spectre de biofilms, dans divers types d'installation et ne nécessitant pas de précautions particulières d'emploi, visant autant les installations de type cuve que les installations de type tuyauterie. Le détergent élimine une partie superficielle du biofilm et mouille et/ou gonfle les structures organiques du biofilm grâce au caractère dispersant de l'agent dispersant présent dans le composant détergent. Ceci favorise donc l'accessibilité du composant enzymatique qui fragilise et dégrade la matrice du biofilm. Cette action combinée des trois types d'enzyme et du composant détergent favorise l'accessibilité à la composition des couches plus profondes et permet un détachement optimal de tout type de biofilm tout en préservant le substrat.

De préférence, ledit au moins un composant enzymatique comprend une proportion de protéase(s) comprise entre 10 et 50%, une proportion de laccase(s) comprise entre 5 et 35% et une proportion de polysaccharidase(s) comprise entre 5 et 20% en poids par rapport au poids total du composant enzymatique, les 100% du composant enzymatique étant éventuellement atteints à l'aide d'un excipient ou solvant conventionnel, par exemple un alcool.

Selon un mode de réalisation préféré de l'invention, le composant enzymatique peut contenir entre 1 et 10 protéases, préférentiellement entre 1 et 5 protéases, plus préférentiellement il peut contenir 2, 3, 4 ou 5 protéases.

Des exemples non limitatifs d'enzymes protéases appartenant à la classe EC 3.4 et susceptibles d'être utilisées dans l'invention sont les amino-peptidases (EC 3.4.11), les dipeptidases (EC 3.4.13), les dipeptidyl-peptidases et tripeptidyl-peptidases (EC 3.4.14), les peptidyl-dipeptidases (EC 3.4.15), les sérine carboxypeptidases (EC 3.4.16), les métallo carboxypeptidases (EC 3.4.17), les cystéine carboxypeptidases (EC 3.4.18), les oméga peptidases (EC 3.4.19), les sérine endopeptidases (EC 3.4.21), les cystéine endopeptidases (EC 3.4.22), les aspartique endopeptidases (EC 3.4.23), les métallo endopeptidases (EC 3.4.24), les thréonine endopeptidases ((EC 3.4.25), et les endopeptidases appartenant à la classe EC 3.4.99.

Préférentiellement, les protéases appartiennent à la classe EC 3.4.21. Les protéases sont disponibles commercialement et sous différentes formes incluant les poudres, les granulés, les suspensions, les solutions liquides.

Les laccases utilisées dans l'invention appartiennent à la classe EC 1.10.3.2. Les laccases sont des enzymes contenant du cuivre et ont pour fonction d'oxyder un substrat en présence d'oxygène. Plus spécifiquement, les laccases sont des oxydoréductases qui fonctionnent avec l'oxygène moléculaire comme accepteur d'électrons.

La au moins une polysaccharidase utilisée dans l'invention est une enzyme ayant pour fonction de briser des liaisons au sein des polysaccharides. Préférentiellement, la au moins une polysaccharidase peut être une alpha-amylase, cellulase, hemi-cellulase, glucosidase, beta-glucanase ou pectinase.

Plus préférentiellement, la au moins une polysaccharidase peut être une alpha-amylase appartenant à la classe EC 3.2.1.1, ayant pour fonction de briser des liens (1-4)-alpha-glycosidiques dans des polysaccharides contenant trois unités ou plus alpha-(1-4)-D-glucose.

Préférentiellement, le composant enzymatique peut comprendre une proportion de laccase(s) approximativement de 30%, une proportion de protéase(s) approximativement de 30%, une proportion d'alpha-amylase(s) approximativement de 10% en poids par rapport au poids total du composant enzymatique, les 100% du composant enzymatique étant éventuellement atteints à l'aide d'un excipient ou solvant conventionnel.

Selon un autre mode de réalisation préféré, si le composant enzymatique comprend 2 protéases, la proportion de laccase(s) peut être approximativement de 30%, la proportion totale de protéases approximativement de 30%, la proportion d'alpha-amylase(s) approximativement de 10% en poids par rapport au poids total du composant enzymatique, les 100% du composant enzymatique étant éventuellement atteints à l'aide d'un excipient ou solvant conventionnel.

Par exemple, le rapport entre chaque protéase peut être compris entre 1:2 et 2:1, préférentiellement le rapport entre chaque protéase peut être de 1:1. Les enzymes présentes dans le composant enzymatique ont une action complémentaire sur le biofilm. Par exemple, la laccase présente une grande efficacité sur les souillures non attaquées par l'alpha-amylase ou les protéases.

Selon un mode de réalisation préféré de l'invention, le composant enzymatique peut être une solution ou sous forme solide.

Préférentiellement, le composant enzymatique est une solution dont le pH peut être compris approximativement entre 8 et 10. Préférentiellement, le composant enzymatique est une solution aqueuse dont le pH peut être compris approximativement entre 8,5 et 9,5 ; plus préférentiellement le pH peut être approximativement de 9,0, et ceci pour préserver au maximum l'intégrité des enzymes.

Alternativement, le composant enzymatique peut être sous forme solide tel que par exemple sous forme d'un lyophilisat, de poudres, de granulés ou sous tout autre forme permettant la solubilisation dudit composant dans un solvant, puis il sera ultérieurement dissous dans ledit solvant. Le solvant peut être de l'eau ou une solution aqueuse, acide, basique, alcoolique, tamponnée ou neutre. Le composant enzymatique solubilisé pourra alors dans ce cas être ultérieurement dilué dans une solution aqueuse contenant éventuellement un ou plusieurs composés tels que par exemple des détergents pour former la solution de nettoyage.

Dans une forme de réalisation avantageuse selon l'invention, ledit au moins un composant détergent comprend une proportion d'agent séquestrant comprise entre 1 et 10% en poids par rapport au poids total du composant détergent, ce qui représente un optimum entre efficacité, stabilité et coût.

L'agent séquestrant est une substance chimique ayant la capacité à former des complexes avec des ions minéraux qu'il fixe sous une forme empêchant leur précipitation par les réactions habituelles. A titre d'exemple, l'agent séquestrant peut être l'acide éthylène-diamine-tétraacétique, le glucono-delta-lactone, le gluconate de sodium, le gluconate de potassium, le gluconate de calcium, l'acide citrique, l'acide phosphorique, l'acide tartrique, l'acétate de sodium, le sorbitol, un composé comportant un atome de phosphore. Préférentiellement, l'agent séquestrant peut être un oxyde de phosphore tel qu'un phosphonate, un phosphinate ou un phosphate ou leur mélange, ou un sel de celui-ci, une amine ou un oxyde d'amine portant au moins, dans sa structure, un groupement fonctionnel phosphine, oxyde de phosphine, phophinite, phosphonite, phosphite, phosphonate, phosphinate ou phosphate, seul ou en combinaison, ou un sel de ceux-ci.

Plus préférentiellement, l'agent séquestrant peut être un phosphonate ou un sel de celui-ci, une amine ou un oxyde d'amine comportant au moins, dans sa structure, un groupement fonctionnel phosphine, oxyde de phosphine, phosphinite, phosphonite, phosphite, phosphonate, phosphinate ou phosphate, seul ou en combinaison, ou un sel de ceux-ci. A titre d'exemple non limitatif, le phosphonate peut être de formule générale R¹(R²O)(R³O)P=O dans lequel R¹, R² et R³ représentent indépendamment un groupe hydrogène, alkyle, alkyle substitué, alkyle-amino substitué ou non, aminoalkyl substitué ou non, aryle ou aryle substitué. A titre d'exemple non limitatif, l'amine ou l'oxyde d'amine peuvent comporter un, deux ou trois substituant(s) de formule générale CR⁴R⁵W dans laquelle R⁴ et R⁵ représentent indépendamment l'un de l'autre un groupe hydrogène, alkyle, alkyle substitué, alkyle-amino substitué ou non, aminoalkyl substitué ou non, aryle ou aryle substitué, et W représente un groupe phosphonate, phosphinate ou phosphate. L'agent séquestrant peut être sous la forme d'un sel de sodium, calcium, lithium, magnésium ou potassium ; préférentiellement, l'agent séquestrant peut être sous la forme d'un sel de sodium, calcium ou potassium.

Dans une variante avantageuse selon l'invention, le composant détergent comprend une proportion d'agent dispersant comprise entre 1 et 10% en poids par rapport au poids total du composant détergent.

L'agent dispersant est donc une substance chimique ayant la capacité à améliorer la séparation des particules d'une suspension afin de prévenir l'agglutination, l'agrégation et/ou la décantation. L'agent dispersant peut être un polymère soluble ou partiellement soluble dans l'eau tel que par exemple le polyéthylène glycol, les dérivés de la cellulose ou un polymère comprenant au moins un motif acide acrylique ou ester acrylique. Préférentiellement, l'agent dispersant est un polymère comprenant au moins un motif acide acrylique ou ester acrylique de formule générale -(CH₂-CH-COOR)- dans lequel R représente un groupe hydrogène, alkyle ou alkyle substitué, aryle ou aryle substitué. En particulier l'agent dispersant est un polymère ayant un poids moléculaire moyen Mw compris approximativement entre 500 et 10000.

Plus préférentiellement, l'agent dispersant est un polymère de l'acide acrylique. En particulier, l'agent dispersant peut être un homopolymère de l'acide acrylique ayant un poids moléculaire moyen compris approximativement entre 2000 et 6000.

Dans une autre forme de réalisation selon l'invention, le composant détergent comprend une proportion d'agent mouillant comprise entre 1 et 15% en poids par rapport au poids total du composant détergent.

L'agent mouillant est une substance chimique amphiphile, ou une composition comprenant ladite substance chimique amphiphile, qui modifie la tension superficielle entre deux surfaces. L'agent mouillant a pour avantage de favoriser l'étalement d'un liquide sur un solide. L'agent mouillant peut être anionique, cationique, non-ionique ou zwitterionique. Préférentiellement, l'agent mouillant peut être un mouillant anionique ou non-ionique, c'est-à-dire que la partie hydrophile est chargée négativement ou ne comporte aucune charge nette, ou peut être une composition comprenant un mouillant anionique. Plus particulièrement, l'agent mouillant peut être un ester de saccharose ou une composition comprenant un alkyle sulfate de sodium et un alcool.

Avantageusement, et de préférence, dans le composant détergent selon l'invention, ledit agent mouillant est non moussant à chaud et est de préférence choisi dans le groupe des sulfate d'alkyle sodique en C₆ à C₁₀, les sulfates d'alcool éthérés en C₆ à C₁₀, les sulfonates d'alkylearyles en C₆ à C₁₀.

Le fait que l'agent mouillant soit un agent mouillant non moussant à chaud permet une utilisation dans les installations comportant de nombreuses tubulures et tuyaux, évitant de cette façon la formation de mousse, sans altérer, bien au contraire, les performances tensioactives et/ou émulsionnantes de la composition selon l'invention. Il est bien entendu que l'apport d'une solution détergente efficace sans génération de mousse limite les étapes de rinçage, ce qui est particulièrement souhaitable, surtout dans les installations à tubulures ou tuyaux multiples.

Comme pour le composant enzymatique, le composant détergent peut être sous forme solide à dissoudre dans un solvant et/ou dans une phase aqueuse ou sous forme liquide.

Lorsqu'il est sous forme solide, il peut soit être mis directement en solution dans la solution formée par le composant enzymatique éventuellement déjà dilué dans la phase aqueuse, soit être mis en solution dans un solvant, préalablement à sa dilution dans la solution formée par le composant enzymatique et la phase aqueuse, ou dans la phase aqueuse directement, avant la dilution du composant enzymatique.

Lorsque le composant détergent est sous forme liquide, les 100% du composant détergent sont éventuellement atteints généralement à l'aide d'eau et, préalablement à l'application sur le biofilm, il sera dilué dans une phase aqueuse, contenant éventuellement déjà le composant enzymatique.

Par les termes "mise en solution" au sens de la présente invention, on entend soit une mise en solution d'un composé solide dans une phase liquide (solvant), soit une dilution d'un composé liquide dans une autre phase liquide avec lequel le composé liquide est miscible.

Alternativement, selon l'invention, les étapes b) et c), ou b') et c'), peuvent être effectuées de manière simultanée pour former une solution de ladite composition selon l'invention.

Selon un mode de réalisation préféré de l'invention, le pH de la solution formée lors de l'étape b) est compris approximativement entre 11,0 et 14,0, préférentiellement compris approximativement entre 12,0 et 14,0 et plus préférentiellement compris entre 12,8 et 13,8.

Selon l'invention, le pH de la solution de ladite composition formée à l'étape c) ou c') est compris entre 6,5 et 7,5 et une solution basique est ajoutée suite à ladite application d) de la solution de ladite composition sur ledit substrat pendant ladite période de temps prédéterminée, de façon à effectuer un saut de pH jusqu'à environ 8 à 9.

De cette façon, lorsque la composition selon l'invention est appliquée sur le substrat, le pH régnant est compris entre 6,5 et 7,5, ce qui permet à la laccase d'être dans des conditions optimales d'activité. Ensuite, le fait d'effectuer un saut de pH jusqu'à environ 8 à 9 permet aux autres enzymes d'avoir à leur tour leur efficacité optimale. De cette façon, elles auront aussi à leur tour des conditions d'activité optimales, ce qui a pour résultat que le biofilm sera éliminé de manière particulièrement avantageuse puisque chaque enzyme présente aura des conditions optimales pour effectuer son action et le biofilm sera détaché et éliminé complètement. En outre, puisque les composants basiques sont généralement utilisés dans les installations dans lesquelles un biofilm peut se former, il n'y a pas d'ajout de composant exogène qui pourrait être problématique pour valider l'étape de nettoyage. Par exemple, pour sanitiser une installation, il est courant d'appliquer de la soude et donc, l'apport de substance de nature différente est limité dans le cadre de la présente invention.

De préférence, la température de la solution du composant détergent formée lors de l'étape b) ou c') peut être comprise approximativement entre 35°C et 50°C. Selon un mode de réalisation préféré de l'invention, la composition selon l'invention est appliquée sur un substrat couvert d'un biofilm pendant approximativement 30 à 50 minutes, ce qui représente un temps d'application relativement court pour une telle efficacité.

La présente méthode permet d'éliminer efficacement la totalité ou la quasi-totalité du biofilm, ne laissant alors sur le substrat que des cellules isolées sans protection de la matrice. L'action postérieure d'un biocide permet de détruire la souche microbienne. Une phase de désinfection ultérieure sera donc beaucoup plus efficace suite à l'application d'une solution de la composition selon l'invention que suite à l'application d'une phase de nettoyage conventionnelle ne permettant pas cette élimination totale de la matrice.

Ainsi, selon un mode de réalisation préféré de l'invention, la méthode comprend en outre une étape ultérieure d'application d'un biocide. Par exemple, les biocides peuvent être, de manière non limitative, de type oxydant tel que l'acide peracétique, le peroxyde d'hydrogène, le monopersulfate de potassium, l'hypochlorite de soude. Selon l'invention, l'application d'un biocide doit être postérieure à l'application de la composition selon l'invention pour éviter la désactivation par les biocides des enzymes présentes dans ladite composition.

D'autres formes de réalisation du procédé selon l'invention sont mentionnées dans les revendications annexées.

D'autres caractéristiques, détails et avantages de l'invention ressortiront de la description donnée ci-après, à titre non limitatif et en faisant référence aux dessins annexés et aux exemples.
La FIG. 1 représente un graphique montrant la quantité de cellules d'une souche de *Pseudomonas fluorescens* présente sur un substrat après l'application de différentes solutions de traitement.
La FIG. 2 représente un graphique montrant la quantité de cellules d'une souche *Bacillus mycoïdes* présente sur un substrat après l'application de différentes solutions de traitement.
La FIG. 3 représente un graphique montrant la quantité de cellules d'une souche *Bacillus cereus* présente sur un substrat après l'application de différentes solutions de traitement.
La FIG. 4 représente des images de la surface d'un substrat, prises par un microscope à épifluorescence, après l'application de différentes solutions de traitement sur ledit substrat recouvert par un biofilm de la souche de *Pseudomonas fluorescens.*
La FIG. 5 représente des images de la surface d'un substrat, prises par un microscope à épifluorescence, après l'application de différentes solutions de traitement sur ledit substrat recouvert par un biofilm de la souche de *Bacillus mycoïdes.*
La FIG. 6 représente des images de la surface d'un substrat, prises par un microscope à épifluorescence, après l'application de différentes solutions de traitement sur ledit substrat recouvert par un biofilm de la souche de *Bacillus cereus.*
LA FIG.7 illustre les résultats de colorations de biofilms avant nettoyage par des solutions de nettoyage (A) et après nettoyage par lesdites solutions (B).
La FIG. 8 est un graphique présentant l'efficacité de quatre enzymes vis-à-vis de biofilms provenant de la souche *Chryseobacterium meningosepticum*
La FIG. 9 est un graphique présentant l'efficacité de quatre enzymes vis-à-vis de biofilms provenant de la souche *Bacillus Cereus.*
La FIG. 10 est un graphique présentant l'efficacité de quatre enzymes vis-à-vis de biofilms provenant de la souche *Pseudomonas fluorescens.*

Sur les figures, les éléments identiques ou analogues portent les mêmes références.

Notons que les figures et les exemples repris ci-après n'illustrent pas le procédé suivant l'invention mais portent sur l'effet des solutions appliquées pour l'élimination des biofilms présents sur un substrat.

### Exemple 1.-

Description de la sélection des conditions de test : les tests sont effectués dans le cadre d'une procédure de nettoyage en place (NEP) sur un pilote industriel. Les biofilms sont développés sur une face plane d'un cylindre en inox perforé en son centre. Ce cylindre, une fois introduit dans une canalisation de diamètre intérieur égal au diamètre extérieur du cylindre, provoque un brusque rétrécissement du diamètre de celle-ci, forçant le liquide à passer par la perforation en son centre. Cette contrainte entraîne des perturbations du flux, créant ainsi des zones mortes à la surface du cylindre. Ces zones mortes sont favorables à la formation d'un biofilm et de plus défavorables à son arrachement mécanique par le flux. Elles représentent donc typiquement des zones difficiles pour l'élimination d'un biofilm.

Trois souches distinctes sont utilisées : *Pseudomonas fluorescens* (fourni par University of Cornell, Department of Food science, Ithaca, New York, 14853, USA (Kathryn J. Boor, kjb4@cornell.edu)), Bacillus mycoides (fourni par le laboratoire AFSSA à Maison-Alfort, France (Brigitte Carpentier, b.carpentier@lerpac.afssa.fr)), *Bacillus cereus* (fourni par INRA, UR638, Processus aux Interfaces Et Hygiène des Matériaux, Villeneuve d'Ascq, France (Christine Faille, Christine.faille@lille.inra.fr)).

Un milieu de croissance est préparé comme suit : des extraits de viande en poudre (Biokar) sont dissous dans l'eau distillée à 0,1% et sont stérilisés. 20 ml d'inoculum à 5.10⁷ CFU/ml dans une solution de Trypticase Soy broth (TSB, Biokar) sont déposés sur la surface des cylindres. Ces cylindres sont incubés en chambre humide à 30°C pendant 2 heures pour permettre l'adhésion des cellules. Ensuite la solution de TSB est retirée et remplacée par 20 ml du milieu de croissance viande et les cylindres sont incubés 24 h à 30°C. Le milieu de croissance est alors remplacé par du milieu frais à base viande et les cylindres sont à nouveau incubés pendant 24h.

La procédure de nettoyage consiste à introduire les cylindres contaminés par le biofilm dans les canalisations droites d'un pilote industriel nettoyé par circulation (procédure de nettoyage en place - NEP). Les solutions de traitement sont circulées durant 30 minutes à 45°C avec un débit de 300 l/h. Pour deux des souches, des essais ont été également réalisés avec un débit de 600l/h. Chaque essai est répété trois fois et une moyenne est établie.

Le développement et l'élimination des biofilms sont suivis par observation microscopique et par quantification des cellules viables présentes sur les cylindres par dénombrement sur milieu Trypticase Soy Agar après décrochement des cellules.

### Description du protocole de préparation de la composition

Le composant détergent est préparé en mélangeant dans un volume d'eau déterminé un phosphonate, un polyacrylate et un mouillant anionique. Les proportions respectives dans le composant détergent sont de 3%, 4% et 3%. Le pH de la solution est amené à 13,3 par dilution. Une solution du composant enzymatique est préparée. Celle-ci comprend une proportion de 30% de protéases (EC 3.4.21), 30% de laccase (EC 1.10.3.2) et 10% d'alpha-amylase (EC 3.2.1.1) et un excipient conventionnel.

Le pH de la solution du composant enzymatique est porté à 9 par ajout progressif d'une solution d'hydroxyde de potassium. La solution de la composition selon l'invention est préparée par l'ajout dans de l'eau du composant détergent et du composant enzymatique. La solution de ladite composition selon l'invention comprend 1% de composant détergent et 0,05% de composant enzymatique. Le pH de la solution de ladite composition selon l'invention était approximativement de 10.

### Description des tests et de leurs résultats

### Test avec la souche de Pseudomonas fluorescens

Le graphique représenté à la FIG. 1 montre la quantité de biofilm présente sur la paroi du cylindre après l'application de solutions de traitements suivant les conditions énoncées ci-dessus. Les surfaces du substrat ont été recouvertes d'un biofilm formé par la souche de *Pseudomonas fluorescens.* La quantité initiale de biofilm présente avant le traitement a été notée A1 dans la FIG. 1.

Trois solutions ont été ensuite comparées : une solution comprenant uniquement de l'eau (B1), une solution de soude à 0,5% (C1) et une solution de la composition selon l'invention (D1). Les solutions ont été appliquées à un débit de 300 l/h et une température de 45°C. La solution (B1) comprenant de l'eau permet d'évaluer l'arrachement mécanique des biofilms par le flux.

On observe ainsi que le biofilm a résisté à l'arrachement mécanique puisque les quantités de biofilm sont identiques (courbes A1 et B1). L'application d'une solution de la composition selon l'invention a permis une réduction importante du biofilm (courbe D1, 10³ CFU) par rapport à une solution de soude (courbe C1, 10⁵ CFU). La composition selon l'invention est donc plus efficace qu'un traitement de référence (solution de soude) sur un biofilm résistant à l'arrachement mécanique.

La FIG. 4 présente plusieurs images de la surface d'un substrat prises au microscope à épifluorescence après l'application des différentes solutions de traitement à 300 l/h. L'image (B4) montre la surface du substrat soumis à une solution d'eau, l'image (C4) montre la surface du substrat soumis à une solution de soude à 0,5% et l'image (D4) montre la surface du substrat soumis à une solution de la composition selon l'invention.

Ces données montrent clairement que la composition selon l'invention a éliminé la totalité de la matrice des biofilms (coloration diffuse), ne laissant sur les surfaces que des cellules isolées ou de petits groupes de cellules réparties sur une seule couche, non protégées par une matrice. La solution de soude n'a quant à elle pas permis l'élimination complète de la matrice (coloration diffuse), qui protège toujours partiellement les cellules résiduelles. Une phase de désinfection ultérieure sera donc beaucoup plus efficace suite à l'application d'une solution de la composition selon l'invention.

### Test avec la souche de Bacillus mycoïdes

Le graphique représenté à la FIG. 2 montre la quantité de biofilm présente sur la paroi du cylindre après l'application de solutions de traitements suivant les conditions énoncées ci-dessus. Les surfaces du substrat ont été recouvertes d'un biofilm formé par la souche de *Bacillus mycoïdes.* La quantité de biofilm présente avant le traitement est repris à la courbe (A2).

Cinq solutions ont ensuite été comparées : une solution comprenant uniquement de l'eau (B2), une solution de soude à 0,5% (C2), une solution de la composition selon l'invention (D2 et F2), et une solution de soude à 2% (E2). Les solutions A2-D2 ont été appliquées à un débit de 300 l/h tandis que les solutions E2-F2 ont été appliquées à un débit de 600 l/h.

Le biofilm de *Bacillus mycoïdes* est relativement sensible à l'arrachement mécanique, sa quantité étant diminué de 90% par l'application de la solution (B2). L'application de la solution de la composition selon l'invention (D2 et F2) a montré des performances équivalentes à la solution de soude (C2 et E2) que le débit soit de 300 l/h ou de 600 l/h.

La FIG. 5 représente plusieurs images de la surface du substrat prises au microscope à épifluorescence après l'application des différentes solutions de traitement à 300 l/h. L'image (B5) montre la surface du substrat soumis à une solution d'eau, l'image (C5) montre la surface du substrat soumis à une solution de soude à 0,5% et l'image (D5) montre la surface du substrat soumis à une solution de la composition selon l'invention.

Ces données montrent clairement que la composition selon l'invention a éliminé la quasi-totalité de la matrice des biofilms, ne laissant que des cellules isolées ou de petits groupes de cellules réparties sur une seule couche, non protégées par une matrice. La solution de soude n'a pas permis une élimination plus efficace du biofilm. L'action postérieure d'une solution désinfectante sera donc plus efficace si une solution de la composition selon l'invention a précédemment été utilisée.

### Test avec la souche de Bacillus cereus

Le graphique représenté à la FIG. 3 montre la quantité de biofilm présente sur la paroi du cylindre après l'application de solutions de traitements suivant les conditions énoncées ci-dessus. Les surfaces du substrat ont été recouvertes d'un biofilm formé par la souche de *Bacillus cereus.* La quantité de biofilm présente avant le traitement est repris à la courbe A3.

Cinq solutions ont été comparées : une solution comprenant uniquement de l'eau (B3), une solution de soude à 0,5% (C3), une solution de la composition selon l'invention (D3 et F3), et une solution de soude à 2% (E3). Les solutions B3, C3, D3 ont été appliquées à un débit de 300 l/h tandis que les solutions E3 et F3 ont été appliquées à un débit plus élevé (600l/h). Un débit plus élevé influence l'arrachement mécanique.

Le biofilm de *Bacillus cereus* résiste totalement à l'arrachement mécanique (courbe A3 et B3). La composition selon l'invention a montré une plus grande efficacité à un débit de 300 l/h par rapport à une solution de soude à 0,5% (courbe C3) et même à un débit de 600 l/h par rapport à une solution de soude plus concentrée (courbe E3).

La FIG. 6 représente plusieurs images de la surface d'un substrat prises au microscope à épifluorescence après l'application des différentes solutions de traitement. L'image (B6) montre la surface du substrat soumis à une solution d'eau, l'image (C6) montre la surface du substrat soumis à une solution de soude à 0,5% et l'image (D6) montre la surface du substrat soumis à une solution de la composition selon l'invention.

Ces images montrent de nouveau que la composition selon l'invention élimine la quasi-totalité de la matrice des biofilms, ne laissant que des cellules isolées ou de petits groupes de cellules réparties sur une seule couche, non protégées par une matrice.

La solution de soude ne permet pas une élimination efficace du biofilm. La phase de désinfection du substrat suivant la phase d'élimination du biofilm sera donc plus efficace après l'application d'une solution de la composition selon l'invention lors de cette phase d'élimination.

Les différents tests effectués dans cet exemple démontrent l'efficacité de la composition selon l'invention pour l'élimination d'un biofilm sur un substrat. La composition selon l'invention se révèle être plus efficace qu'une solution de soude couramment employée dans l'art antérieur. La composition selon l'invention se montre également très efficace pour plusieurs types de biofilm issus de bactéries différentes. La composition selon l'invention apporte donc une solution efficace aux problèmes mentionnés dans l'art et connue de l'homme du métier.

### Exemple 2.-

Des tests ont également été réalisés sur deux lignes de production en industrie (industrie de production de beurre et de margarine). Des coupons en acier inoxydable (8*1cm) sont placés durant 15 jours dans des circuits de production présentant des contaminations sporadiques. Ces coupons sont donc soumis aux cycles de production et de nettoyage standard de ces installations.

La présence de biofilm dans l'installation a été déterminée par le développement d'un biofilm sur les coupons. Une procédure de nettoyage en place spécifique et faisant intervenir la composition selon l'invention a été appliquée dans l'installation, en présence des coupons contaminés, et dont l'efficacité a été démontrée par l'élimination des biofilms formés sur les coupons.

La présence et l'importance des biofilms sur les coupons ont été déterminées en laboratoire par coloration des coupons et comparaison de la coloration obtenue avec une échelle visuelle (méthode HYDROBIO®, BKG), ainsi que par observation au microscope optique (40x et 100x).

Deux protocoles de nettoyage ont été testés : le premier protocole a servi de référence et correspond en l'application de solutions connues dans l'art, le second protocole correspond en l'ajout d'une étape d'application d'une solution de la composition selon l'invention.

En particulier, le protocole I comprend les étapes suivantes : de nettoyage à l'eau, nettoyage alcalin, de rinçage, de sanitation (ou désinfection) et de rinçage final. Le protocole I a été appliqué toutes les 32 heures pendant une semaine. Le nettoyage à l'eau a été appliqué pendant 15 minutes à une température comprise entre 60 et 65°C. Le nettoyage alcalin permet de nettoyer les canalisations de la matière organique présente suite à un cycle de production. Cette étape a été réalisée avec une solution de soude à 3,5% qui a été appliquée pendant 2*15 minutes à une température comprise entre 70-75°C.

L'étape de sanitation permet l'élimination des germes éventuellement encore présents mais non protégés par la matrice du biofilm. Cette étape comprenait l'application d'une solution de Deptil OX à 1,5% (HYPRED) pendant 2*15 minutes à une température comprise entre 20 et 25°C. La solution de Deptil OX contient un mélange d'acide peracétique et de peroxyde d'hydrogène. Les étapes de rinçage ont été effectuées pendant 15 minutes à une température comprise entre 20 et 25°C.

Le protocole II différait du protocole I en ce qu'il comprenait une étape de nettoyage spécifique avant l'étape de sanitation (ou désinfection). En outre, le protocole II n'a été appliqué qu'une seule fois.

L'étape de nettoyage spécifique consistait en l'application d'une solution de la composition selon l'invention. Ce mélange a été appliqué pendant 30 minutes à une température comprise entre 40 et 45°C. Les résultats obtenus sont repris dans le tableau 1.

**Tableau 1**

| | Quantité de biofilm suivant le protocole I (g/m²) | Quantité de biofilm suivant le protocole II (g/m²) |
|---|---|---|
| Ligne de production 1 | 25 | <5 |
| Ligne de production 2 | 25 à 35 | <5 |

Le protocole I n'a pas permis de limiter et d'empêcher la croissance d'un biofilm malgré l'étape de sanitation (désinfection). Le biofilm qui s'est formé sur les coupons en acier inoxydable au coeur de l'installation est donc très résistant. L'application du protocole II incluant une étape de nettoyage spécifique avec une solution de la composition selon l'invention a permis une élimination beaucoup plus efficace de ce biofilm (sous la limite de détection) de ce biofilm. Il a ainsi démontré une efficacité supérieure du nettoyage spécifique qui apporte donc une solution innovante aux problèmes de l'art antérieur.

### Exemple 3.-

Des essais permettant de comparer les optimum de pH ont été réalisés.

Les biofilms sont développés sur des coupons en inox préalablement lavés et stérélisés. Un inoculum bactérien (Pseudomonas aeruginosa) est préparé par culture durant 16h dans un milieu TSB 10% (tryptone soy broth). Cette préculture est diluée pour obtenir une densité optique (DO) de 0,05 à 600nm et 500 µl de cette solution ont été étalés sur chaque coupon. Les coupons sont incubés durant 48h dans des boîtes de pétri maintenues humides et placées en étuve à 30°C. Après 2 heures, la solution bactérienne est remplacée par 500 µl de milieu frais, TSB 10%. Après 24h, ce milieu de culture est renouvelé.

Les coupons sont ensuite nettoyés et sont donc installés sur des tiges métalliques et séparés par des écrous. Puis ils sont placés dans les solutions de nettoyage. Les coupons sont laissés à tremper dans les solutions de nettoyage susdites pendant un temps total de 30 minutes.

Les solutions de nettoyages sont les suivantes :
1. Composition selon l'invention à pH 4,5, trempage pendant 30 minutes
2. Composition selon l'invention à pH 7, trempage pendant 30 minutes
3. Composition selon l'invention à pH 9,7, trempage pendant 30 minutes.

Les coupons ont ensuite été placés dans des tubes à essai contenant 10 ml d'une solution TSB + 0,5% Tween 80 pour le comptage des bactéries. Les tubes à essai ont été incubés pendant 5 minutes sur la table avant d'être soniqués pendant 2 minutes trente et vortexés pendant 30 secondes. Les étapes d'incubation, sonication et vortex ont été répétées.

Les coupons ont ensuite été récupérés et le milieu comprenant la solution TSB + 0,5 % de tween a fait l'objet de dilutions sérielles en utilisant de l'eau peptonée (Eau peptonée exempte d'indole: préparer 10 ml d'une dissolution à 15g/l. Puis diluer 1ml de cette solution dans 1 litre d'eau stérile) et a ensuite été étalé sur boîte de pétri et incubé à 30 °C pendant une nuit avant comptage.

Le biofilm a également été coloré de la manière suivante: les coupons sont égouttés et plongés dans la solution de coloration durant 10 minutes sélectif pour les protéines de la matrice du biofilm. Ensuite, les coupons sont plongés dans les différentes solutions de nettoyage 1 à 3 mentionnées ci-dessus pendant deux fois 10 minutes (les solutions de nettoyage sont remplacées entre les deux étrapes de nettoyage).avant de sécher à l'air libre.

### Résultats :

A pH acide (4,5) : un tapis des bactéries s'est développé sur la surface des boites de Pétri, ce qui indique la présence d'une grande quantité de biofilm résiduel sur les coupons.

A pH neutre (7) : un tapis des bactéries s'est développé sur la surface des boites de Pétri.

A pH alcalin (9,7) : pas des bactéries présentes dans les boites de Petri.

La figure 7 illustre les résultats des colorations des biofilms. La figure 7 A illustre les témoins avant nettoyages par les solutions de nettoyage 1 à 3 tandis que la figure B illustre les coupons d'acier après nettoyage par les solutions 1 à 3 de gauche à droite. Comme on peut le voir, un nettoyage à pH alcalin par la composition selon l'invention est clairement plus efficace qu'à pH acide ou à pH neutre, le biofilm développé sur le coupon est entièrement décroché à pH alcalin alors qu'il persiste aux autres pH.

### Exemple comparatif

Un essai visant à déterminer l'efficacité de quatre enzymes vis-à-vis de trois souches bactériennes a été mené.

Les résultats de cet essai sont présentés aux figures 8 à 10. Des biofilms ont été réalisés sur des coupons (12) en acier conformément à l'exemple 3.

4 coupons ont donc été réalisés pour chaque souche bactérienne *(Pseudomonas fluorescens, Bacillus cereus et Chryseobacterium meningosepticum).* Les coupons ont été ensuite rincés à l'eau avant d'être incubés chacun avec une solution de nettoyage contenant respectivement une première protéase, une deuxième protéase, une laccase et une polysaccharidase pendant 30 minutes.

Le tableau 2 comprend le résultat de comptages bactériens tandis que les figures 8 à 10 présentent en ordonnée le logarithme du résultats des comptages bactériens et en abscisse le type d'enzyme utilisé.

**Tableau 2.-**

| | *Chryseobacterium meningosepticum* | *Bacillus cereus* | *Pseudomonas fluorescens* |
|---|---|---|---|
| Niveau de contamination de départ | 2.10⁸ | 7.10⁶ | 2.10⁸ |
| Première protéase | 4.10⁵ | 7.10² | 1.10² |
| Deuxième protéase | 2.10⁶ | 9.10³ | 3,8.10⁴ |
| Laccase | 8.10³ | 1,3.10⁵ | 1,6.10² |
| Amylase | 1.10⁵ | 9,6.10⁴ | 3,9.10⁶ |

Comme on peut le voir, certaines souches bactériennes sont plus sensibles à l'action de la polysaccharidase et de la laccase, comme c'est le cas de *Chryseobacterium meningosepticum* (Gram-). Le *Bacillus Cereus* (Gram +) est quant à lui plus sensible à la combinaison des deux protéases alors que *Pseudomonas fluorescens* (Gram -) est plus sensible à la combinaison de la première protéase et de la laccase.

Il est bien entendu que la présente invention n'est en aucune façon limitée aux formes de réalisations décrites ci-dessus et que bien des modifications peuvent y être apportées sans sortir du cadre des revendications annexées. Par exemple, les domaines d'application des compositions selon l'invention se sont avérées particulièrement utiles pour une utilisation en trempage, par exemple dans le domaine hospitalier (nettoyage de matériel de laboratoire, de matériel chirurgical), ou par circulation dans des tubulures, comme par exemple dans l'industrie agroalimentaire, dans les installations de conditionnement d'air, dans les installation de potabilisation et désalinisation de l'eau, pour les coques de bateau et tout type de matériel immergé, dans les circuits des machines à lessiver, lave-vaisselle, etc. ou encore par trempage et par circulation dans des tubulures.

### ABREGE

### "PROCEDE D'ELIMINATION DES BIOFILMS"

Procédé pour l'élimination des biofilms présents sur un substrat.

## Revendications

1. Méthode pour l'élimination des biofilms présents sur un substrat **caractérisée en ce qu'**elle comporte les étapes suivantes de :
- a) mise à disposition d'un composant détergent comprenant un agent séquestrant ainsi qu'un agent mouillant et un agent dispersant et d'un composant enzymatique contenant au moins une protéase, au moins une laccase et au moins une polysaccharidase,
- b) mise en solution du composant détergent dans une phase aqueuse,
- c) mise en solution du composant enzymatique dans la solution formée à l'étape b) pour former une solution d'une composition pour l'élimination des biofilms présents sur un substrat, ladite solution formée présentant un pH compris entre 6,5 et 7,5,
ou b') mise en solution du composant enzymatique dans une phase aqueuse,
- c') mise en solution du composant détergent dans la solution formée à l'étape b') pour former une solution pour l'élimination de biofilms, ladite solution formée présentant un pH compris entre 6,5 et 7,5,
- d) application de la solution de ladite la composition formée à l'étape c) ou c') sur le substrat pendant une période de temps prédéterminée, en particulier comprise entre 15 minutes et 4 heures,
- e) une addition d'une solution basique suite à ladite application d) de la solution de ladite composition sur ledit substrat pendant ladite période de temps prédéterminée, de façon à effectuer un saut de pH jusqu'à environ 8 à 9.

2. Méthode selon la revendication 1, **caractérisée en ce qu'**elle comprend en outre une étape ultérieure d'application d'un biocide sur le substrat.

## Patentansprüche

1. Verfahren zur Entfernung von auf einem Substrat vorhandenen Biofilmen, **dadurch gekennzeichnet, dass** es die folgenden Schritte umfasst:
- a) Bereitstellung eines detergierenden Bestandteils, welcher einen Komplexbildner sowie ein Netzmittel und ein Dispergens umfasst, und eines enzymatischen Bestandteils, welcher zumindest eine Protease, zumindest eine Laccase und zumindest eine Polysaccharidase enthält,
- b) Auflösung des detergierenden Bestandteils in einer wässrigen Phase,
- c) Auflösung des enzymatischen Bestandteils in der in Schritt b) gebildeten Lösung, um eine Lösung einer Zusammensetzung zur Entfernung der auf einem Substrat vorhandenen Biofilme zu bilden, wobei die erwähnte gebildete Lösung einen pH-Wert von zwischen 6,5 und 7,5 aufweist,
oder b') Auflösung des enzymatischen Bestandteils in einer wässrigen Phase,
- c') Auflösung des detergierenden Bestandteils in der in Schritt b') gebildeten Lösung, um eine Lösung zur Entfernung von Biofilmen zu bilden, wobei die erwähnte gebildete Lösung einen pH-Wert von zwischen 6,5 und 7,5 aufweist,
- d) Anwendung der Lösung der erwähnten, in Schritt c) oder c') gebildeten Zusammensetzung auf dem Substrat während eines vorbestimmten Zeitraums, insbesondere zwischen 15 Minuten und 4 Stunden lang,
- e) eine Hinzufügung einer basischen Lösung nach der erwähnten Anwendung d) der Lösung der erwähnten Zusammensetzung auf dem erwähnten Substrat während des erwähnten vorbestimmten Zeitraums, sodass ein pH-Sprung auf etwa 8 bis 9 eintritt.

2. Verfahren nach Anspruch 1, **dadurch gekennzeichnet, dass** es überdies einen nachfolgenden Schritt der Anwendung eines Biozides auf dem Substrat umfasst.

## Claims

1. Method for removing biofilms present on a substrate, **characterized in that** it comprises the following steps of:
- a) providing a detergent component containing a sequestrant, a wetting agent and a dispersant and providing an enzymatic component containing at least one protease, at least one laccase and at least one polysaccharidase,
- b) dilution of the detergent component into an aqueous phase,
- c) dilution of the enzymatic component into the solution formed at step b) to form a solution of said composition for removing biofilms present on a substrate, said formed solution having a pH comprised between 6,5 and 7,5,
or -b') dilution of the enzymatic component into an aqueous phase,
-c') dilution of the detergent component into the solution formed at step b') to form a solution for removing biofilms, said formed solution having a pH comprised between 6,5 and 7,5,
- d) applying the solution of said composition formed at step c) or c') on the substrate for a predetermined period of time, in particular from 15 minutes and 4 hours,
- e) addition of a basic solution following to the application d) of the solution of said composition on said substrate during said predetermined period of time, to perform a pH jump up to approximatively 8 to 9.

2. Method according to claim 1, **characterized in that** it further comprises a subsequent step of applying a biocide to the substrate.
